# EUROPEAN PATENT SPECIFICATION

(11) **EP 2 035 545 B1**
(45) Date of publication and mention of the grant of the patent: **04.01.2017**
(21) Application number: 07790114.8
(22) Date of filing: 31.05.2007
(51) Int. Cl.: C12N 5/00, A61F 2/02, A61L 24/04, A61L 24/10, C12N 5/0775, A61L 27/38

(54) **Platelet gel biomembrane for the treatment of skin lesions**
Blutplättchen-Gel-Biomembran zur Behandlug von Hautläsionen.
Piomembrane de gel plaquettaire pour le traitement de lésions cutanées

(30) Priority: 31.05.2006 IT RM20060289
(43) Date of publication of application: 18.03.2009
(73) Proprietor: Biorigen International SA, Lutry (Lausanne) (CH)
(72) Inventor: CANCEDDA, Ranieri, I-16145 GENOVA (IT); MASTROGIACOMO, Maddalena, I-16132 Genova (IT); SCALA, Marco, I-16162 Genova (IT)
(74) Representative: Capasso, Olga
(86) International application number: PCT/IT2007/000382
(87) International publication number: WO 2008/004260

(56) References cited:
- EP-A1- 1 674 115
- WO-A-00/29552
- WO-A-03/008003
- US-A1- 2004 127 832
- CANCEDDA RANIERI ET AL: "Tissue engineering and cell therapy of cartilage and bone." MATRIX BIOLOGY, vol. 22, no. 1, March 2003 (2003-03), pages 81-91, XP009091299 ISSN: 0945-053X
- BABBUSH CHARLES A ET AL: "An in vitro and in vivo evaluation of autologous platelet concentrate in oral reconstruction." IMPLANT DENTISTRY 2003, vol. 12, no. 1, 2003, pages 24-34, XP009100171 ISSN: 1056-6163
- LI W-J ET AL: "A three-dimensional nanofibrous scaffold for cartilage tissue engineering using human mesenchymal stem cells" BIOMATERIALS, ELSEVIER SCIENCE PUBLISHERS BV., BARKING, GB, vol. 26, no. 6, February 2005 (2005-02), pages 599-609, XP004523985 ISSN: 0142-9612
- THOR ANDREAS: "Reconstruction of the anterior maxilla with platelet gel, autogenous bone, and titanium mesh: a case report." CLINICAL IMPLANT DENTISTRY AND RELATED RESEARCH 2002, vol. 4, no. 3, 2002, pages 150-155, XP009091319 ISSN: 1523-0899
- LEONARDI F ET AL: "Hip revision surgery in cases with acetabular bone loss: PPR rings, homologous bone and platelet gel" JOURNAL OF ORTHOPAEDICS AND TRAUMATOLOGY ; OFFICIAL JOURNAL OF THE ITALIAN SOCIETY OF ORTHOPAEDICS AND TRAUMATOLOGY, SPRINGER-VERLAG, MI, vol. 5, no. 2, 1 August 2004 (2004-08-01), pages 73-76, XP009091318 ISSN: 1590-9999
- CANCEDDA ET AL: "A tissue engineering approach to bone repair in large animal models and in clinical practice" BIOMATERIALS, ELSEVIER SCIENCE PUBLISHERS BV., BARKING, GB, vol. 28, no. 29, 10 August 2007 (2007-08-10), pages 4240-4250, XP022192632 ISSN: 0142-9612
- Giovanni Crovetti ET AL: "Platelet gel for healing cutaneous chronic wounds", Transfusion and Apheresis Science, vol. 30, no. 2, 1 April 2004 (2004-04-01), pages 145-151, XP055200056, ISSN: 1473-0502, DOI: 10.1016/j.transci.2004.01.004
- Giovanni Crovetti ET AL: "Platelet gel for healing cutaneous chronic wounds", Transfusion and Apheresis Science, vol. 30, no. 2, 1 April 2004 (2004-04-01), pages 145-151, XP055200071, ISSN: 1473-0502, DOI: 10.1016/j.transci.2004.01.004
- Carolina Weller ET AL: "Editors Wound Dressings Update", Journal of Pharmacy Practice and Research Volume, 1 January 2006 (2006-01-01), page 318, XP055200118, Retrieved from the Internet: URL:http://jppr.shpa.org.au/lib/pdf/gt/GT0 612.pdf [retrieved on 2015-07-03]

## Description

### TECHNICAL FIELD OF THE INVENTION

The present invention relates to an engineered tissue bio-membrane, an implant device, for tissue regeneration and repair as bone reconstruction, repair of lesions of the skin and of soft tissues, e.g. chronic ulcers, difficult wounds, bedsores, chinks, tendon lacerations, soft tissue substance loss, and methods for the production thereof.

### BACKGROUND ART

In clinical practice and in surgery, it is ever more needed to identify a valid system to repair large tissue lesions associated with substance losses.

In orthopedics and maxillofacial surgery, in the past few years a bio-technological approach has been proposed which suggests the use of the patient's own cells in association with ceramic scaffolds, appropriately designed with respect to the lesion to repair large substance losses (Quarto et al., 2001). The results presented in the literature, which are certainly valid, can nonetheless be further improved. Pre-clinical studies and a pilot clinical study have highlighted that the lack of vascularization at the level of the implant itself can lead to cell death (apoptosis), nullifying the real effect of "bone marrow stromal cells", BMSC.

In a recent trial in sheep (Mastrogiacomo et al., 2006), a re-absorbhble ceramic scaffold was used, and it was observed that, starting from periosteum residues, a bone formation. process can be triggered, able to fully repair the lesion. The progressive formation of bone tissue and the filling of the ceramic pores are accompanied by a re-absorption of the ceramic and by the simultaneous vascularization of the implant. Unfortunately, the use of the patient's periosteum is only rarely applicable in reconstructive surgery.

With regard to repair/regeneration of other tissues, the use of techniques like surgical debridement or transplants of vascularized tissue to repair skin lesions due to burns. difficult or post-operation wounds, chronic ulcers, .are .only one_{'} of the few possible applications (Warnke, 2004). Considering the regulatory role in tissue repair that is played by platelets and macrophages, recently the use of local applications of platelet gel has been proposed to repair skin lesions of various kinds (Scala, 2000).

### DESCRIPTION OF THE INVENTION

The authors have now devised a bio-membrane that solves the problems of the prior art and that is able to induce *in vivo*, in animals and in humans, the production of neo-tissue. Said neo-tissue can be bone, when the bio-membrane is implanted as envelope of a scaffold, such as reabsorbable porous ceramic scaffolds for the repair of large size bone deficits. The neo-tissue can also be a soft tissue as in the repair of skin lesions by direct contact.

Therefore, an object of the present invention is a biomembrane constituted by a platelet gel comprising a platelet concentrate, thrombin and calcium gluconate, said biomenbrane being covered by a patch of hyaluronic acid for use in the treatment of skin lesions.

The bio-membrane of the invention is advantageously usable if partially dehydrated before its application.

For the repair of skin lesions, the invention proposes an adhesive plaster The adhesive plaster is constituted by three essential elements: the pad, the support and the adhesive. The pad can be constituted by cotton mixed with acrylic with high absorption capacity or by a material with similar characteristics and covered by a thin film of polyester or by a material with similar characteristics, loaded with platelet gel rich in active biological factors which, in contact with the wound, accelerates healing. A further object of the invention is an adhesive plaster **comprising the biomembrane as defined above being covered by a patch of hyaluronic acid for use in the treatment** of skin lesions.

The present invention will now be described in its non limiting examples, referring to the following figures:

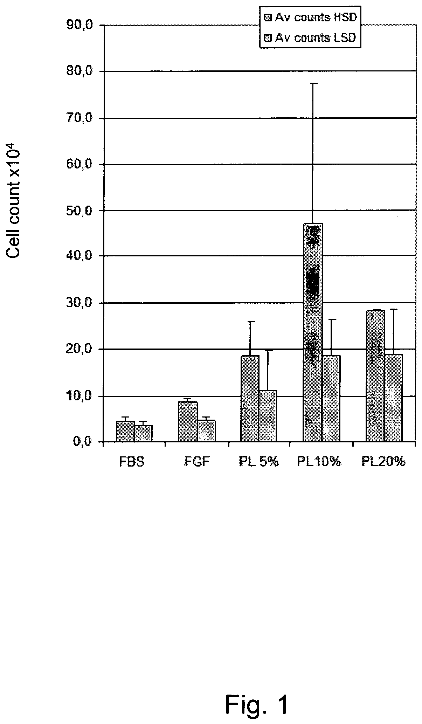

- Figure 1. Histogram of the cell proliferation of human BMSC in the presence of Platelet Lysate (PL) (5%, 10%, 20%), FBS 10% or FGF-2 1 ng/ml. Proliferation was evaluated by cell count of wells plated at low cell density (LSD, Low seeding density) and high cell density (HSD, high seeding density).

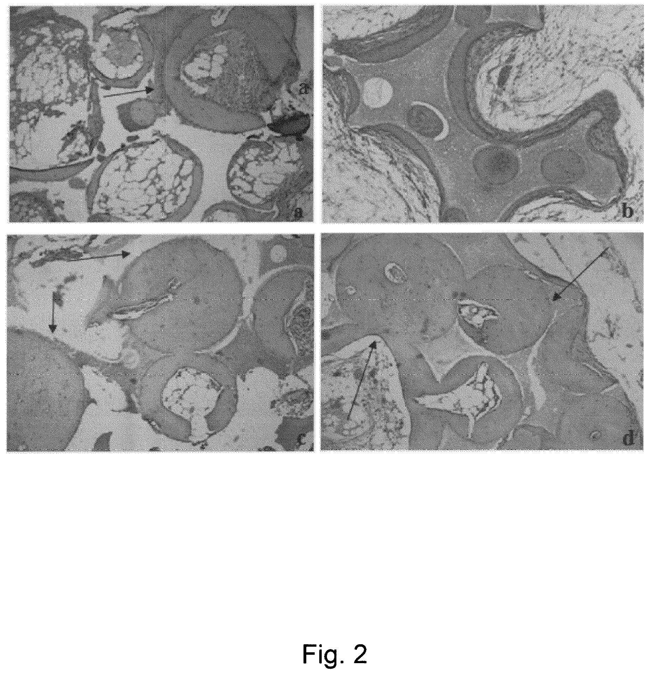

- Figure 2. Bone tissue formation. A film of platelet gel associated with sheep BMSC was wrapped around a cube of hydroxyapatite (HA, 100% pure HA - 60-70 mm³) and implanted subcutaneously in immunodeficient mice for 4 and 8 weeks: the cells were bridled within the matrix of the gel (IN) (panels a and c) or layered on the surface of the gel (ON) (panels b and d). Bone tissue formation is highlighted by the hematoxylin-eosin staining indicated by the arrows.

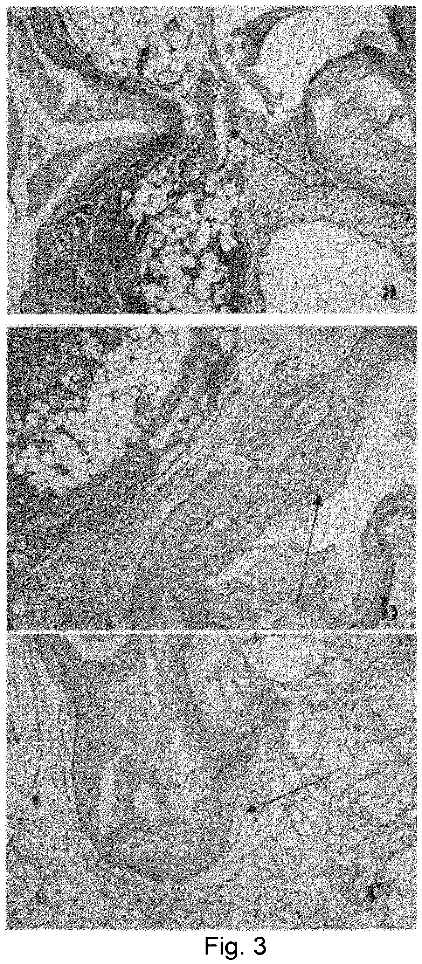

- Figure 3. Bone tissue formation. A film of platelet gel alone (a) or associated with sheep BMSC IN (b) or ON (c) was wrapped around skelite® (TCP-HA - 2000-2500 mm³) scaffolds and implanted in immunodeficient mice for 8 weeks. Bone tissue formation is highlighted by the hematoxylin-eosin staining indicated by the arrows.

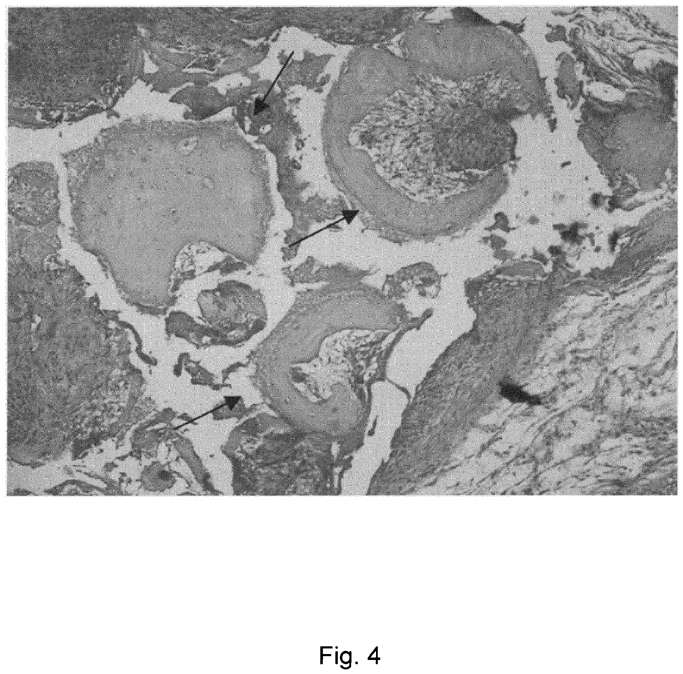

- Figure 4. Bone tissue formation. A film of platelet gel with sheep BMSC on cubic scaffolds (100% pure -64mm³). The BMSCs were layered on the surface of the platelet gel and stimulated with osteogenic medium for two weeks. Hematoxylin-eosin staining highlights bone tissue formation in the ceramic pores, as indicated by the arrows.

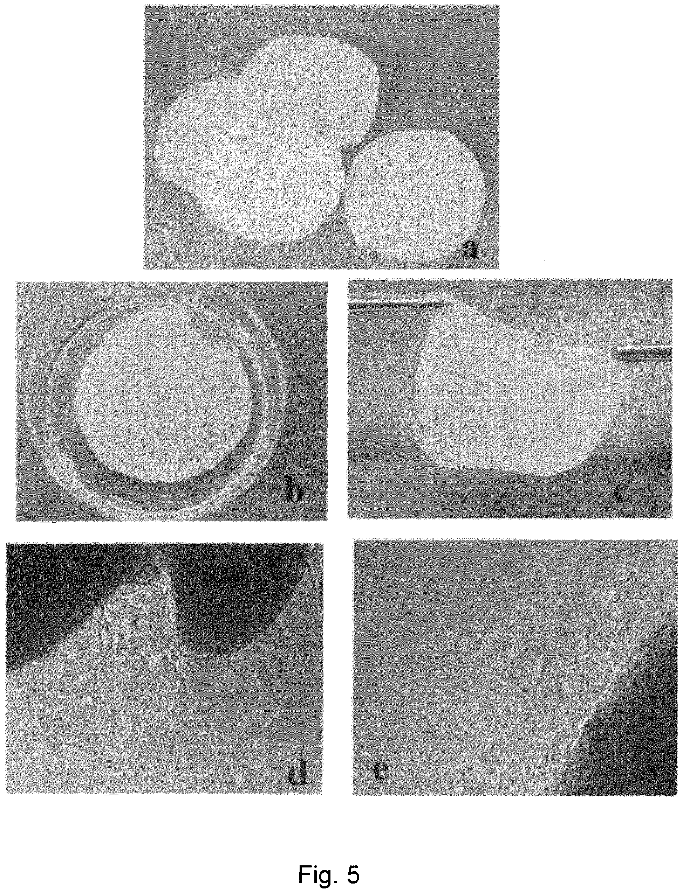

Figure 5. Dehydration of the bio-membrane. The bio-membrane is dehydrated by means of sterile absorbent paper (a) assuming a consistency and elasticity that enable easily to transpose it into the implant site (b-c). Cell vitality tests demonstrate that the vitality of the cells included in the bio-membrane after dehydration (e) is equal to that of the non dehydrated control.

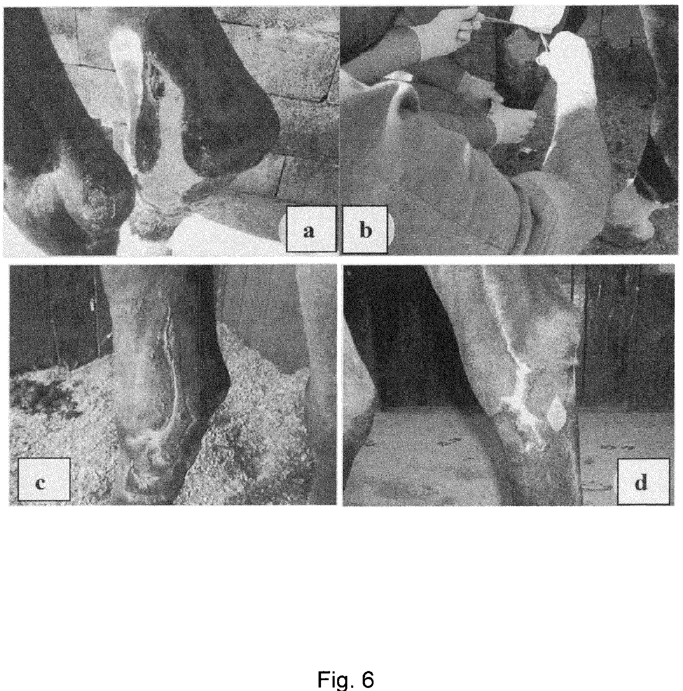

-Figure 6. Repair of a skin lesion in a horse. A bio-membrane constituted by autologous horse platelet gel and hyaluronic acid patch was layered on the lesion.

### Cell Cultures

Bone Marrow Stromal Cells (BMSC) were isolated from human or sheep bone marrow. The samples, after authorization by the patient or by the ethical board in the case of trials on animals, were drawn from the iliac crest (10 ml).

In some experiments, cells were derived directly from human or sheep periosteum biopsies by successive digestions with 0.25% of Collagenase according to standard protocols.

The bone marrow was washed in PBS and the nucleate cell count per ml of sample was. performed. Part of the sample was plated at very low density (100 ml/plate) to evaluate the number of CFU in F12 medium supplemented with 2mM glutamine, 100 U/ml penicillin and 100 µg/ml streptomycin, 1 ng/ml FGF-2 and 10% of ^{;}bovine fetal serum. The remaining part of the marrow aspirate was destine to the expansion of the cells in culture in standard culture medium. When the cells reached the first confluence, they were trypsinized and plated on Petri dishes or on platelet gel in the surface (method called IN), or associated to the platelet gel during its polymerization (method called ON). The concentration of the plated cells in the IN or ON gel varies from 1x10⁶ to 6x10⁶ cells per cm² of surface area.

### Preparation of Human Platelet Gel

The human platelet gel was obtained from blood components prepared by the Transfusion Center of the San Martino Hospital in Genoa. From the withdrawal of peripheral blood of the human or sheep donor, the following are obtained:
a) a *cryoprecipitate* containing coagulation factors and immunoglobulins;
b) a *platelet concentrate* (CP) containing platelets;
c) *autologous thrombin* that intervenes in the polymerization process of fibrin.

The preparation of the individual components proceeds as follows:
The blood is centrifuged for 7 minutes at 20°C at 1700g/min and allows the separation of a platelet rich plasma called PRP.

The PRP is centrifuged at 4400g/min for 5 minutes at 20°C allowing the separation of the platelet poor plasma called PPP and platelet concentrate (CP). The CP is frozen and thawed to ambient temperature at the time of use.

The PPP is frozen at -40°C and thawed at 4°C throughout the night in satellite sack. When thawing is complete, the cryoprecipitate is obtained by siphoning.

The CP and the cryoprecipitate were mixed in plate in a 1:1 ratio. 1 ml of autologous thrombin and 1 ml of 10% calcium gluconate on a total volume of 10 ml were added to initiate the gel polymerization process.

To assess the effect of the platelet gel on human BMSG, the cells were glown in the presence of culture medium complete with supplements and with different concentrations of Platelet Lysate (LP) (5%, 10% and 20%), obtained from the CP, as described below. The cells were plated in wells at high density (10,000 cells/well) and at low concentration (2,000 cells/well) in the presence or absence of LP. Cell proliferation was evaluated, in the different conditions, by cell count when the culture had reached semiconfluence (10 days). For the preparation of the Platelet Lysate, the protocol described by Doucet C et al. (2005) was followed. The LP is obtained after subjecting the CP to 3 freezing/thawing cycles to promote complete platelet lysis and total release of all growth factors contained therein (PDGF-bb, PDGF-aa, EGF, IGF etc...) and in the presence of low EDTA concentration. The LP was added to the culture at different concentrations.

### Preparation of Platelet Gel from an Animal (Horse)

The day before the intervention, two units of 450 ml of blood are drawn from the horse, by means of a standard triple bag for the withdrawal of human blood containing ACD ((citric acid + sodium citrate + dextrose) as an anticoagulant (Fresenius HemoCare CODE T2375). The bags were centrifuged in an ALC PM980R centrifuge (BTCase, Italy) for 8 minutes at 500 x g ; the blood is then separated into red cells and Platelet Rich Plasma (PRP), partially entering into the Buffy-Coat®.

The PRP must be re-centrifuged at 5,000 x g for 7 minutes to obtain the Platelet Concentrate (CP) that must be re-suspended in about 80 mL of autologous plasma adjusting platelet count between 0.5 and 3x10⁶ microliter.

The bag containing the CP is placed in an agitator thermostated at + 22° until the time of use.

The day of the intervention, the CP is drawn under sterile hood from the bags, with syringes labeled with the identifying data of the horse.

### The CP is ready to be injected into the site of the lesion to be repaired

If the product is to be used in the form of semi-solid gel, at the time of use some sterile plastic Petri dishes of about 10 cm diameter are prepared adding 10 mL of platelet concentrate, 1 mL of Calcium Gluconate (ind. Farmaceutica Senese, Italy, Lot. No. After a few minutes, the transformation occurs from fibrinogen to fibrin with "gelification" of the Platelet Concentrate which can be applied topically on large surfaces.

In case of tendon lesions, the product will be injected non gelified into the site of the lesion, under echographic guidance.

### Bone Tissue Formation in Vivo

To evaluate bone tissue formation *in vivo,* a small animal model was used, i.e. the immunodeficient mouse (Nu/Nu strain or SCID strain). Ceramic scaffolds of different sizes and breakdown (Engipore®, 100 % HA, Finceramica, Faenza, Italy and Skelite®, TCP70/HA30, Millenium Biologix) were implanted subcutaneously into the back of immunodeficient mice after enveloping them with a bio-membrane of platelet gel and human or sheep BMSC.

The BMSC were layered on the gel (ON) or included in the gel (IN) directly during the polymerization phase. The bio-membrane of platelet gel (obtained with the ON method or with the IN method) was kept in complete medium but without FGF-2 for 1-3 days, before being enveloped around cubic scaffolds (60-70 mm³) of HA 100%, (EngiPore®). In some experiments, a few minutes before the implant, the sample was enveloped by an additional membrane of fresh platelet gel without cells, to assure a greater supply of growth factors. In each animal, 4 scaffolds were implanted including a control implant, in which the BMSC were loaded directly into the scaffold using fibrin glue (Tissucol®, Baxter) as an adjuvant of the adhesion of the cells to the ceramic.

In a second series of experiments, larger size (hollow cylinders of 2000-2500 mm³) reabsorbable ceramic scaffolds made of skelite® (TCP 70%, HA 30%, Millenium Biologix, Ontario, Canada) were used. In this case, a single sampler was implanted per animal.

In some experiments, the platelet gel conjugated to BMSC was partially dehydrated by superposing absorbent, sterile filter paper, thereby forming a more consistent and more easily handled bio-membrane. In the partially dehydrated gel, the cells proliferated normally, maintaining their osteogenic potential after implant in the animal.

In some experiments, the platelet gel conjugated to human or sheep BMSC was pre-treated *in vitro* with osteogenic medium. 24 hours after preparation, the platelet gel membranes were transferred in culture medium supplemented with factors inducing osteogenic differentiation: 10⁻⁸ M dexamethasone, 10 mM b-glycerol-phosphate (BGP), and 50 mg/ml enveloped around HA cubes, re-enveloped by fresh platelet gel without BMSC and implanted subcutaneously in ID mice.

*In vivo* implants were retrieved after 4 and/or 8 weeks and subjected to histological analysis: the samples were decalcified and enclosed in paraffin. The sections were Hematoxylin-Eosin stained according to standard procedures.

### Skin Lesion Repair (Horse)

The bio-membrane, constituted by platelet gel prepared as indicated above, was layered on the lesion and covered by a patch of hyalurohic acid (ComvaTec Hyalofill, FAB Srl, Abano Terme, Italy) or by other material with coverage, characteristics such as OpSite Flexigrid (Smith and Nephew). To the biological implant was then applied a modestly compressive traditional medication with gauze and bandages replaced after 14 days. The follow up was conducted by clinical monitoring of the patient and measuring the surface area of the remaining lesion at regular time intervals after the start of the treatment.

### RESULTS

### Proliferation

In the first phase of the work, the effect of the platelet gel on human BMSC was assessed, growing the cells in the presence of culture medium supplemented with serum only, FGF-2 only or with 3 different concentrations of LP. The cells were plated in wells at low or high density. The chart shown in Fig. 1 shows that the cells grown at high, or low density in the presence of 5% LP proliferate significantly more than cells grown in serum only Cells grown in the presence of FGF-2 also exhibit less proliferation than those treated with LP. z High BMSC proliferation is observed in medium supplemented with 5,10 or 20% LP The addition of LP determines a significant increase in proliferation with respect to the conditions with serum only or FGF-2. While the activity peak is obtained with 10%, in subsequent *in vitro* experiments the LP concentration used was 5%, because it is equally eflicient.

### In Vivo Differentiation

Human or sheep BMSC were loaded at the surface of the gel (ON- method) or directly, in the gel mesh (IN method) forming a veritable compact film, called bio-membrane, which was used to envelop a small or large ceramic scaffold.

In Fig. 2, platelet gel bio-membranes prepared with BMSC both with the IN method and with the ON method were enveloped around cubes of 100% HA and implanted for 4 and 8 decalcified samples, paraffin-enclosed samples, it was possible to observe that the cells, both enmeshed in the gel (IN, a,c) and kept on the surface of the gel (ON, b,d) are able to differentiate into osteoblasts and to deposit osteogenic matrix into the ceramic, pores already during the first four weeks of implant. A significant line of osteoblasts at the edge of the newly laid bone indicates an intense bone matrix laying activity. After 8 weeks implant, a greater quantity of bone fills the pores of the ceramic. No significant difference was observed in the formation of bone tissue both in the samples enveloped by bio-membranes with layered cells in the surface (IN method) and in those with bio-membranes with cells enmeshed in the fibrin mesh (ON method).

Though the model of the ID mice is one of the most accredited *in vivo* models to test the osteogenicity of cells and biomaterials, the authors deemed it appropriate to repeat the experiment under test conditions that would more closely approach the "real" conditions to be found in clinical practice.

For this purpose, a porous, reabsorbable ceramic scaffold was used (Mastrogiacomo et al., 2006), with a greater presence of Tricalcium phosphate and a smaller presence of hydroxyapatite (TCP 70%, HA 30%). Hollow cylinders of about 2,000 mm³ were enveloped with platelet gel bio-membranes, alone or associated with cells, with the IN method or with the ON method and implanted in ID mice for 8 weeks. Panel a) in Fig. 3 shows no bone tissue formation in samples enveloped by platelet gel without cells. Only fibrous tissue together with fatty tissue populates the pores of the ceramic. However, some vascularization can be observed, confirming the important role played by the platelet gel in the vascularization of the lesion site (Rhee JS, 2004). When the scaffold is wrapped by film of platelet gel associated with cells obtained with the IN method) or with the ON method (Fig. 3b-c), bone tissue is observed in the pores of the ceramic. Abundant osteoblasts are distributed at the surface of the laid bone. Upon microscopic observation of the ample colored section, it is possible to note a distribution of newly formed bone tissue that goes from the periphery to the center of the scaffold.

In the attempt to generate a bio-membrane with the highest, osteogenic and angiogenic potential starting from a bio-membrane of platelet gel and BMSC (human or sheep) obtained both with IN method and with ON method the bio-membranes were pre-trcated. *in vitro* for a period of 2 weeks with osteoinductive culture medium (see methods), to promote an initial laying of matrix prior to the transfer on scaffold. After 8 weeks of *in vivo* implant, the samples (Fig. 4) exhibited good bone tissue formation distributed from the periphery to the center of the samples.

The prolonged maintenance of BMSC in platelet gel *in vitro,* reduces their osteogenic potential. However, the bio-membranes can be pre-treated with osteoinductive medium for a period of two weeks, assuring the maintenance of the full osteogenic potential.

In Fig. 5 we show the dehydration of the bio-membrane by means of a continuous superposition of disks of sterile absorbent paper that completely removes the soluble part of the membrane and water. This procedure generates a membrane that is more elastic and easier to handle during the surgical procedure without altering the vitality of the cells included therein.

With respect to the repair of skin lesion, an example of treatment of skin lesion in a horse is reported (Fig. 6). In all treated animals, it was sufficient to apply the platelet gel once to trigger the regenerative process (Fig. 6a-b). The figure clearly shows the reduction of the lesion at 15 days from the treatment (Fig. 6 c) and *restitutio ad integrum* after thirty days (Fig. 6d) when the horse presume its sports-competition activity.

### BIBLIOGRAPHY

1) Quarto et al., N. Engl. J. Med., 2001, 344 (5):385-86.
2) Mastrogiacomo et al., , Tissue Eng. 2006, 12(5):1261-73.
3) Rhee et al., Thromb Haemost. 2004, 92(2):394-402.
4) Scala M et al., Proc. Am Acad Maxillofacial Prosth. International Congress Maxillofacial Prosthetics in the 21st Century, Kauai - Hawaii; November 11-14,2000: 132.
5) Warnke PH et al., Lancet 2004, 364: 766-70.

## Claims

1. A biomembrane constituted by a platelet gel comprising a platelet concentrate, thrombin and calcium gluconate, said biomembrane being covered by a patch of hyaluronic acid for use in the treatment of skin lesions.

2. An adhesive plaster comprising the biomembrane being covered by a patch of hyaluronic acid as claimed in claim 1 for use in the treatment of skin lesions.

## Patentansprüche

1. Biomembran, die von einem Thrombozytengel gebildet ist, welches ein Thrombozytenkonzentrat, Thrombin und Calciumglukonat umfasst, wobei die Biomembran von einem Hyaluronsäurepflaster bedeckt ist, zur Verwendung bei der Behandlung von Hautläsionen.

2. Klebepflaster, umfassend die Biomembran, die von einem Hyaluronsäurepflaster nach Anspruch 1 bedeckt ist, zur Verwendung bei der Behandlung von Hautläsionen.

## Revendications

1. Biomembrane constituée d'un gel plaquettaire comprenant un concentré plaquettaire, de la thrombine et du gluconate de calcium, ladite membrane étant recouverte d'un patch d'acide hyaluronique pour une utilisation dans le traitement de lésions cutanées.

2. Pansement adhésif comprenant la biomembrane étant recouverte d'un patch d'acide hyaluronique selon la revendication 1 pour une utilisation dans le traitement de lésions cutanées.
